# EUROPEAN PATENT SPECIFICATION

(11) **EP 0 652 436 B1**
(45) Date of publication and mention of the grant of the patent: **27.03.2002**
(21) Application number: 94308135.6
(22) Date of filing: 04.11.1994
(51) Int. Cl.: G01N 33/497, A61B 5/00, C12Q 1/00

(54) **Bio-sensor for measuring alcohol concentration, method for manufacturing the bio-sensor and drunkometer using the same**
Biosensor zum Messen der Alkoholkonzentration, Verfahren zur Herstellung des Biosensors, und den Biosensor benutzendes Betrunkenheitsmessgerät
Biodétecteur pour la mesure de la concentration d'alcool, méthode pour la fabrication du biodétecteur, et appareil pour la détermination du degré de l'ivresse utilisant ce biodétecteur

(30) Priority: 04.11.1993 KR 9323296
(43) Date of publication of application: 10.05.1995
(73) Proprietor: LG ELECTRONICS INC., Seoul (KR)
(72) Inventor: Park, Je Kyun, Songpa-ku, Seoul (KR); Lee, Hee Jin, Kwangmyung-si, Kyungki-do (KR)
(74) Representative: Cross, Rupert Edward Blount

(56) References cited:
- EP-A- 0 357 027
- EP-A- 0 537 761
- CA-A- 2 050 057
- US-A- 4 897 173
- BIOSENSORS & BIOELECTRONICS, vol. 7, no. 10, 1992, pages 701-708, XP000614094 T. D. GIBSON: "EXTENDED SHELF LIFE OF ENZYME-BASED BIOSENSORS USING A NOVEL STABILISATION SYSTEM"
- PATENT ABSTRACTS OF JAPAN vol. 13, no. 169 (C-587), 21 April 1989 & JP-A-63 317096 (MATSUSHITA ELECTRIC IND. CO. LTD.), 26 December 1988,

## Description

### Background of the Invention

The present invention relates to a drunkometer for measuring alcohol concentration using a bio-sensor for reacting with a vapor-phase alcohol gas.

A drunkometric technology using a conventional bio-sensor for measuring alcohol concentration is described as follows.

First, in the case of a gas measuring bio-sensor using conventional electrochemical measuring methods, living bodies such as enzymes or microbes are immobilized in a membrane form on the surface of an electrode. The immobilized living bodies and the electroactive material generated by their reaction with a simple enzyme or multistage enzymes are measured using a device such as H₂O₂ electrode, oxygen electrode, NH₄⁺-selective electrode, ion-selective electrode field effective transistor, thereby detecting gases.

Meanwhile, the U.S.Patent No. 4,655,880 disclosed a bio-sensor by which various kinds of oxidase are immobilized on a thick film amperometric electrode by which electroactive material can be measured.

However, the bio-sensor using such electrochemical measuring methods has a drawback in that it should be used in the favorable condition for a bio-reaction. That is to say, such bio-sensors can detect only a liquid-phase gas.

To overcome such a drawback of the conventional bio-sensors, a gas measuring bio-sensor which can measure a vapor-phase organochemical material and the method therefor are disclosed in the Korean patent application No.93-13482.

This technology is about the method for manufacturing a bio-sensor having a structure wherein an induced film having a hydrogel layer on which an enzyme reacting with vapor-phase organochemical material is immobilized, on the electrode of an amperometric device.

Meanwhile, most of drunkometers are being commercially produced using a gas sensor adopting an oxide semiconductor, e.g., TG 822 sensor manufactured by Figaro Inc. of Japan. Examples of the drunkometers include an alcohol checker manufactured by Figaro Inc. of Japan and a breath alert manufactured by Breath Alert MFG of U.S.A.

Such drunkometric technologies are to measure alcohol concentration contained in the gases generated during human's exhalation in the range from 0.1mg/l to 0.8mg/l, thereby displaying a drinking degree using a light emission diode (LED). There has been also proposed a technology by which an alert sound is produced if a drinking degree is higher than or equal to a predetermined level.

The ground for measuring the drinking degree depends on an experimental report that alcohol concentration contained in lml blood almost equals to that contained in 200ml exhalation, that is, the ground for the correlation between alcohol concentration in the exhaled gas and that in blood.

Therefore, since blood alcohol concentration is indirectly known by measuring alcohol concentration of respired gases after drinking, thereby determining the drinking degree.

In order to determine a probable drinking driving which is basis for cancellation of a driver's license as well as a penal prosecution, under the Korean existing laws, there is a need for a sensor for measuring exactly alcohol concentration contained in exhaled gases in the range of 20 to 500 ppm.

Meanwhile, a method for measuring a drinking degree using an enzymatic reaction is disclosed in the Japanese publication laid-open patent Nos. showa 60-196198 and showa 60-172298. These technologies are to measure alcohol concentration contained in aqueous solution, e.g., saliva, using a strip-type test paper, thereby measuring the drinking degree. The international patent WO88/01299 proposed a drinking measuring technology for measuring alcohol concentration contained in gases generated during respiration, by the color change of the test paper.

However, there has not yet been proposed a technology for measuring a drinking degree using a bio-sensor.

The aforementioned conventional technologies for measuring alcohol concentration involve the following problems.

Firstly, the conventional drunkometers using an oxide semiconductor gas sensor adopting an alcohol reactive metal oxide such as SnO₂, TiO₂ or RuO₂ have no selectivity for ethanol. That is to say, in general, those drunkometers are considerably affected by combustible gases like automobile exhaust, LPG, cigarette smoke, or thinner.

Secondly, in the case when drinking degrees are intended to be measured consecutively for a short time of period, if alcohol concentration for a previous person is thick enough to affect on the next person's alcohol concentration to be measured considerably, the exactness of measurement decreases. To avoid affect by the measured result for the previous person, those drunkometers should be used in a predetermined time once after they are used, which disables to measure the drinking degrees for lots of people for a short time of period.

Thirdly, the conventional drunkometers are readily affected by ambient temperature and have serious measuring errors depending on measuring methods. Thus, the drunkometers should be calibrated every two or three months.

Fourthly, most of drunkometers are required to blow up a strong breath into the inlet of the sensor not less than three seconds for measuring the drinking degree. In such a case, saliva may be intermixed into the sensor, which makes the drinking degree to be measured unreliable. Also, the mixed saliva may cause a trouble to the sensor.

Fifthly, since the conventional technology for evaluating the drinking degree using the enzymatic reaction should detect the color change by means of a material such as 2,6-dichloroindophenol, the measured drinking degree may vary depending on the measurers, which causes a lack of objectivity in measuring a drinking degree. Further, there is a trouble in that extinction should be measured again for more precise measurement of a drinking degree.
It would be desirable to solve the following technological problems.
1. In a bio-sensor using an electrochemical principle, in order to measure a vapor-phase sample, i.e., detectable gas, the induced film of the bio-sensor should provide an electrode system. The conventionally proposed gas measuring bio-sensor utilized a hydrogel for measuring a vapor-phase sample.
2. Electrons should be transmitted easily between an enzyme induced film immobilized on the upper portion of the sensor and the electrode so that sufficient electrical signals are generated during the reaction with the vapor-phase sample. For this purpose, an electrode manufacturing technology, an enzyme processing technology and the combination of those technologies should be realized.
3. The drunkometer using a disposable bio-sensor should have excellent individual characteristics of the sensors so that the objectivity of the measured values can be secured. Conventionally, the amperometric devices using thick film forming technology have been manufactured. However, in this case, in order to form an enzyme immobilized layer, since enzyme solution should be dropped on the upper portion of the sensors individually, mass production is difficult and the excellent individual characteristics of the devices cannot be obtained.
4. Since bio-sensors should be continuously provided separately from the drunkometer, the storing capacity of the bio-sensors should be sufficient in view of the distribution period of the bio-sensors.
5. The method for measuring a drinking degree should be simplified using a portable drunkometer and bio-sensor.

### Summary of the Invention

It would be desirable to provide a bio-sensor for measuring alcohol concentration, having a selectivity for ethanol and using an electrochemical principle, which can measure the drinking degree by reacting electrically with vapor-phase alcohol contained in exhaled gas and the method therefor.

It would also be desirable to provide a bio-sensor for measuring alcohol concentration, by which alcohol concentration measured with respect to a previous person does not affect on that measured with respect to the next person and precise measurement can be obtained due to being free from saliva or change of ambient temperatures, and the method therefor.

It would also be desirable to provide a bio-sensor which can measure the drinking degree quantitatively, allow mass production, and have excellent individual characteristic, and the method therefor.

It would also be desirable to provide a bio-sensor which can allow a mass production by forming and printing enzyme paste having a constant viscosity such that a thick film amperometric device which can measure electroactive material is manufactured and enzyme immobilized layer is then formed on a working electrode, and the method therefor.

It would also be desirable to provide a drunkometer using a biosensor having a selectivity for ethanol and using electrochemical principle, which can measure the drinking degree by reacting electrically with vapor-phase alcohol contained in exhaled gas.

It would also be desirable to provide a drunkometer using a biosensor, by which alcohol concentration measured with respect to a previous person does not affect on that measured with respect to the next person and precise measurement can be obtained due to being free from saliva,or change of ambient temperatures.

It would also be desirable to provide a drunkometer for measuring a drinking degree precisely, using a biosensor which can measure the drinking degree quantitatively, allow a mass production, and have excellent individual characteristics.

It would also be desirable to provide a drunkometer which can measure a drinking degree by a simple manipulation and treatment, display the measured drinking degree quantitatively, and endow the objectivity to the measured drinking degree.

Accordingly, the present invention provides a breath alcohol analyzer
for measuring alcohol concentration comprising a biosensor for reacting with a vapor-phase alcohol gas; a sensor and amplifying circuit for sensing and amplifying the current generated from the reaction of the biosensor with the alcohol gas; an analog-to-digital converter for converting a signal output from said sensor and amplifying circuit into a digital signal; a microprocessor for processing said digital signal output from said analog-to-digital converter and determining a drinking degree corresponding to the digital signal, the microprocessor outputting a signal indicating the drinking degree; and a display for receiving the signal output from the microprocessor and displaying the drinking degree.

A bio-sensor for measuring alcohol concentration suitable for use in embodiments of the present invention comprises:
an insulating substrate;
a thick film amperometric device formed on the insulation substrate, which has a plurality of conductive line and connective pads and a plurality of electrodes;
an enzyme immobilized layer formed on one electrode among the plurality of electrodes of amperometric device, on which an enzyme paste is printed;
an outer membrane formed on the substrate having the plurality of electrodes, for forming an electrode system; and
an insulating membrane formed on the substrate excluding the outer membrane.

Such a bio-sensor may be manufactured using a method which comprises the steps of:
manufacturing an enzyme paste;
forming a thick film amperometric device on an insulating substrate;
forming an enzyme immobilized layer by printing the enzyme paste on amperometric device; and
printing and forming an outer membrane on the electrode of amperometric device.

A drunkometer embodying the invention comprises:
a sensor & amplifying circuit for sensing the current generated due to the reaction of the bio-sensor with alcohol and amplifying the generated current;
an analog-to-digital converter for converting a signal output from the sensor & amplifying circuit into a digital signal;
a microprocessor for processing the digital signal output from analog-to-digital converter and turning the same into a drinking degree; and
a display for receiving the signal output from the microprocessor and displaying the drinking degree.

A drunkometer embodying the invention also comprises:
a bio-sensor which reacts with a vapor-phase alcohol sensor;
a microprocessor for signal-processing the current detected by the bio-sensor and converting the processed current into alcohol concentration;
a display for displaying alcohol concentration value of the microprocessor;
a main body in which the microprocessor and bio-sensor are installed;
a cover connected with the main body by means of a hinge, which can be open or shut;
a bio-sensor placement portion opposed to the cover, for placing the bio-sensor to the inner surface of the main body;
a sample gas inlet formed on the side surface of the cover for blowing a testee's exhalation therein;
a sample gas path for guiding the gas passed through the sample gas inlet so as to pass the sensitive portion of the bio-sensor; and
a sample gas outlet for discharging the remaining gas passed through the sample gas path.

### Brief Description of the Drawings

FIG.1 shows a screen printing process for manufacturing a bio-sensor suitable for use in embodiments of the present invention;
FIG.2 is a plan view of a bio-sensor manufactured by the process shown in FIG.1;
FIG.3 is a cross-sectional diagram of the bio-sensor along a line A-A' shown in FIG.2;
FIG.4 is a constitutional example of an apparatus for evaluating the characteristic of the bio-sensor.
FIG.5 is a graph showing an ethanol to chronoamperometric responsive characteristic among the characteristics of the bio-sensor.
FIG.6 is a graph showing an ethanol to current between working electrode and counter electrode among the characteristics of the bio-sensor.
FIG.7 is a block diagram showing the circuit constitution of a portable drunkometer embodying the present invention;
FIG.8 is a detailed diagram of a sensor & amplifying circuit in the portable drunkometer shown in FIG.7;
FIG.9 is a perspective view of a portable drunkometer embodying the present invention;
FIG.10 is a perspective view shown by opening the cover of a portable drunkometer embodying the present invention; and
FIG.11 is a cross-sectional diagram of a bio-sensor according to an embodiment of the present invention.

### * Explanation of reference numerals for essential parts in the drawings

1: Insulating substrate
2: Connective pad
3: Working electrode
4: Counter electrode
5: Reference electrode
6: Insulating layer
7: Enzyme immobilized layer
8: Outer membrane
9: Bio-sensor
10: Measuring cell
11 and 12: 3-way valve
13: Phosphate buffer solution
14: Ethanol standard solution
15: Constant temperature oven
16: Computer
17: Potentiostat
18: Sensor & amplifying circuit
19: Analog-to-digital converter
20: Constant voltage circuit
21: Microprocessor
22: Display
23: Main body
24: Cover
25: Sample gas inlet
26: Sample gas outlet
27: Bio-sensor placement portion
28 and 29: Bio-sensor connective pad
30: Sample gas path

### Detailed Description of Preferred Embodiments

As known well, in the case of a bio-sensor using an electrochemical measuring method, an electrolyte should exist for measuring a vapor-phase organochemical material. In order to analyze a liquid-phase sample such as blood, since the base ion existing in the sample solution functions as an electrolyte, there is no problem in forming an electrode system. However, in order to analyze a vapor-phase sample, solid electrolyte should be used, or electrolyte layer should exist in a liquid phase.

However, the solid electrolyte such as a zirconia cannot be used like an enzyme. Also, in the case of forming the electrolyte in a liquid phase, it is very difficult to form the electrolyte on an immobilized enzyme membrane.

For this purpose, the enzyme induced membrane was conventionally formed by using a hydrogel which can endow functions as a carrier and an electrolyte to the enzyme. However, since this technology has a trouble in that a constant amount of enzyme solution should be dropped individually on the upper portions of sensors, as described above, it is difficult to achieve the excellent individual characteristic and the mass production.

Therefore, after manufacturing a thick film amperometric device which can measure electroactive material such as 1,4-dihydronico thinamide adenine dinucleotide (NADH), using a carbon paste, an enzyme paste having a constant viscosity is printed to then be printed in such a manner that an enzyme immobilized layer is formed on a working electrode.

Also, a paste having a hydroxyethylcellulose as an outer membrane is manufactured and then is printed, thereby improving the effect of absorbing moisture included in the sample gas to be measured while using the sensor.

According to this method, when measuring a vapor-phase alcohol, an electrode system can be formed easily only with the moisture contained in the sample gas. Also, the bio-sensor can be kept in the state of a dry enzyme membrane. Thus, the reduction of enzyme activity can be prevented in keeping the bio-sensor.

In other words, since moisture of about 80mg is contained in the exhaled gas during a human respiration, aforementioned method is adopted for measuring alcohol concentration using the human's exhalation, thereby eliminating the difficulty of forming an electrode system only with the moisture contained in the sample gas.

Therefore, after dry-packing the bio-sensor, the bio-sensor reacts only on measuring, thereby improving its stability for heat and enlogating its life span. Also, since the signal-to-noise ratio is small, differently from another type bio-sensor used in an aqueous phase, and a high enzyme affinity for the vapor-phase organochemical material can be utilized, it is possible to analyze a low concentrated sample which is difficult to measure in a liquid phase.

Also, a carbon is used for a working electrode, which makes the electrode absorption of the enzyme excellent. Enzyme paste composed of carbon powder, high polymers or enzymes, is screen-printed to then form an enzyme immobilized layer. Thus, a mass production becomes advantageous by incorporating the whole processes for manufacturing a bio-sensor into the process for forming a thick film amperometric device, including an enzyme immobilization process.

In the case of utilizing high polymer material such as polyvinyl chloride (PVC), polycarbonate or polyester as an insulating substrate, instead of alumina substrate, a unit of a sensor device can be manufactured at a low cost. In the present invention, the polyester substrate which is most excellent in a paste adhesiveness for manufacturing electrodes and a printing effect, is used. However, in view of the characteristic of high polymers, a paste which requires a sintering condition of a high temperature of about 800°C cannot be used, but a high polymer thick film ink which exhibits electrode characteristic only by sintering at about 150°C or less, is used.

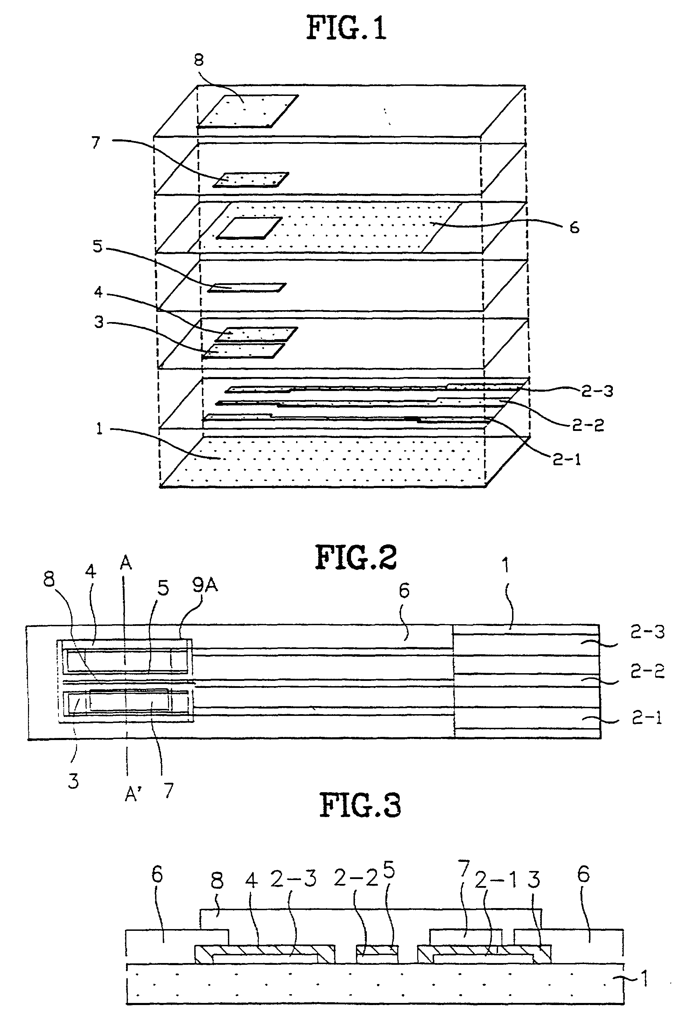

FIG.1 is a perspective view showing a process for manufacturing a bio-sensor for use in embodiments of the present invention. The method for manufacturing the bio-sensor chiefly includes the steps of manufacturing an enzyme paste, forming a thick film amperometric device having an electrode on an insulating substrate, forming an enzyme immobilized layer 7 by printing the enzyme paste on amperometric device, and printing and forming an outer membrane 8 on the electrode of amperometric device.

The step of forming amperometric device includes the steps of screen-printing and forming conductive line and connective pads 2 on the insulating substrate 1, forming an electrode in which current flows on the conductive line and connective pads 2 when it reacts with alcohol in the exhaled gas, and forming an insulating layer 6 on the whole surface of the substrate excluding the electrode.

### 1. Manufacturing an enzyme paste

Carbon powder was used as an enzyme paste material. The diameter of the used carbon powder is 5 micron and the purity thereof is 99.9%. Alcohol dehydrogenase extracted from yeast, a protein whose activity is 400U/mg, is used as an enzyme, and β-nicotinamide adenine dinucleotide (NAD⁺) is used as a cofactor.

NAD⁺ of 450mg and alcohol dehydrogenase of 150mg are sufficiently mixed with 2% hydroxyethylcellulose of 4.29ml cotaining 6% ethylene glycol in a mortar, thereby forming a homogenous enzyme solution.

Carbon power of 1.29g is completely mixed with the homogenous enzyme solution using the mortar, thereby obtaining an enzyme paste. The obtained enzyme paste is refrigerated.

### 2. Screen-printing

Referring to FIG.1, as a substrate, an insulating substrate 1 formed of polyester, having a thickness of 0.3mm and a dimension of 86mm×84mm is prepared. Silver (Ag) paste is screen-printed on the insulating substrate 1 and is dried at the temperature of 110°C for 10 minutes, thereby forming a plurality of conductive line and connective pads 2-1, 2-2 and 2-3.

The carbon paste is screen-printed and the working electrode 3 and counter electrode 4 are formed in parallel on one side of the conductive line and connective pads 2-1 and 2-3. Subsequently, silver (Ag) paste including AgCl is screen-printed, thereby forming a reference electrode 5 on the conductive line and connective pad 2-2 between the working electrode 3 and counter electrode 4.

Dielectric paste is screen-printed on the substrate excluding the portion on which an outer membrane of the upper portion of the electrode is to be formed and the other side of the substrate, and an ultraviolet ray of 80W/cm capacity is irradiated at a velocity of 9m/min, thereby forming an insulating layer 6. Thereby, a thick film amperometric device is completed. In such a process, 20 thick film amperometric devices are obtained on the insulating substrate.

Finally, 6% hydroxyethylcellulose paste is printed on one side of the substrate including the respective electrodes to then form an outer membrane 8, thereby obtaining a bio-sensor.

FIG.2 is a sectional view of a bio-sensor manufactured in accordance with aforementioned processes.

FIG.3 which is a sectional view along a line A-A' shown in FIG.2, shows the sensitive portion of the bio-sensor.

Referring to FIGs.2 and 3, the bio-sensor for measuring alcohol concentration includes an insulating substrate 1, a thick film amperometric device formed on the insulation substrate 1, which has a plurality of conductive line and connective pads 2 and a plurality of electrodes 3 to 5, an enzyme immobilized layer 7 formed on one electrode among the plurality of electrodes of amperometric device, on which an enzyme paste is printed, an outer membrane 8 formed on the substrate having the plurality of electrodes, for forming an electrode system, and an insulating membrane 6 formed on the substrate excluding the outer membrane 8.

Aforementioned amperometric device has a configuration such that three conductive line and connective pads 2 are formed in parallel on the insulating substrate 1, a working electrode 3 and a counter electrode 4 are formed on one side of the substrate on which first and third conductive line and connective pads 2-1 and 2-3 among the plurality of conductive line and connective pads 2 are formed, respectively, and that a reference electrode 5 is formed on one side of the substrate on which a second conductive line and connective pad 2-2 is formed. At this time, the working electrode 3 and counter electrode 4 are formed so as to have a larger width than the first and third conductive line and connective pad 2-1 and 2-3 being in the lower portion thereof, and the reference electrode 5 is formed so as to have the same width as the second conductive line and connective pad 2-2 being in the lower portion thereof. The enzyme immobilized layer 7 is formed only on the upper portion of the working electrode 3.

Referring to FIG. 2, the sensitive portion 9A of the bio-sensor is the portion where the working electrode 3, counter electrode 4, reference electrode 5 and enzyme immobilized layer 7 are printed. The respired gas reacts with the sensitive portion 9A, and then the current corresponding to electrons generated in the sensitive portion 9A flows, The current flowing between the electrodes is detected, thereby measuring the drinking degree.

### 3. Packing an ethanol bio-sensor

As described above, the respective screen-printed sensors are cut and are then airtightly packed, thereby completing the packing of the bio-sensors.

The individual bio-sensor packed separately is provided for measuring the drinking degree.

The bio-sensor manufactured by such a series of processes is evaluated in the following method.

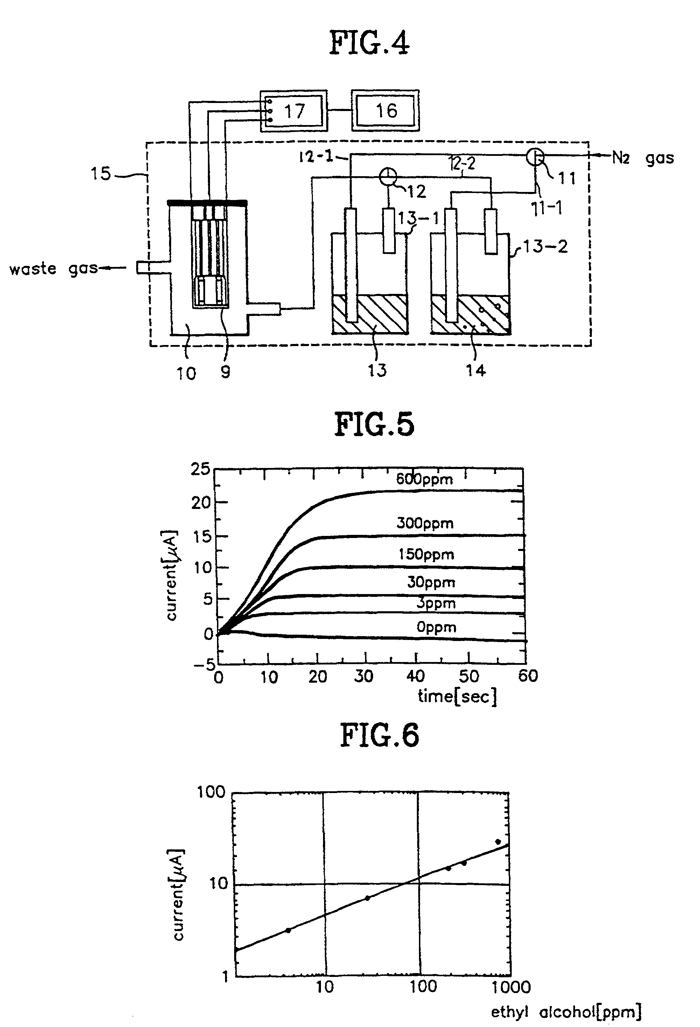

FIG.4 is a constitutional diagram of an apparatus for evaluating the characteristics of the bio-sensor according to the present invention.

Referring to FIG.4, apparatus for evaluating the characteristics of the bio-sensor 9 which evaluates the characteristics of the bio-sensor by using a sample extraction, is placed in a 25ml measuring cell 10. Apparatus is equipped with two 3-way valves 11 and 12 for controlling the supply path of a nitrogen gas. 0.1M phosphate buffer solution and ethanol standard solution are placed in containers 13-1 and 13-2, respectively. The containers 13-1 and 13-2 are connected to Teflon tubes 11-1 and 12-1 for supplying nitrogen gas, respectively. The measuring cell 10, two 3-way valves 11 and 12, 0.1M phosphate buffer solution 13, and ethanol standard solution 14 are provided in a constant temperature oven 15.

Also, a constant differential of electric potential between the working electrode and reference electrode of the bio-sensor 9 is maintained in the measuring cell 10. Potentiostat 17 for detecting the current flowing from the working electrode to the counter electrode and a computer 16 for setting the operational condition of the potentiostat 17, processing the detected information value and producing the characteristics of the bio-sensor are provided outside the constant temperature oven 15.

In apparatus for evaluating the characteristic of the bio-sensor shown in FIG.4, EG & G manufactured by Princeton Applied Research Corp. is adopted as the potentiostat 17 for all electrochemical measurements for evaluating the characteristics of the bio-sensor 9.

The method for evaluating the characteristics of the bio-sensor using aforementioned apparatus will now be described.

Bio-sensor 9 is installed in a 25ml measuring cell and the temperature of the constant temperature oven 15 is adjusted. Nitrogen gas is flown into the container 13-1 containing 0.1M phosphate buffer solution 13 through the Teflon tube 12-1 at a constant velocity, using 3-way valves 11 and 12, thereby activating the bio-sensor 9 being in the measuring cell 10.

Next, the differential of electric potential of the bio-sensor 9 with respect to the reference electrode made of Ag/AgCl is made to be 650mV, using potentiostat 17 connected to the computer 16. Subsequently, nitrogen gas is transmitted into the container 13-2 containing a constant amount of ethanol standard solution 14 of a predetermined density, using the 3-way valves 11 and 12, thereby making the vaporized ethanol gas react with the bio-sensor 9. At this time, the density of the ethanol gas of the internal measuring cell 10 wherein the bio-sensor 9 is installed is calibrated by a gas chromatography.

As the result of the experiment, the chronoamperometric response for the ethanol gas vaporized at a temperature of 25°C is shown as in FIG.5.

Referring to FIG.5, when the differential of electric potential is 650mV, absorption of the ethanol molecules into the enzyme immobilized layer 7 generates an NADH which is an electroactive material, by action of the immobilized enzyme. The generated NADH is oxidized into NAD+ on the working electrode 3 and current flows from the working electrode 3 to the counter electrode 4 at the same time. At this time, the current flowing from the working electrode 3 to the counter electrode 4 is proportionate to the density of the ethanol.

The ethanol concentration can be obtained by using a steady state current or by measuring an initial rate.

FIG.6 shows a standard quantitative curve for ethanol concentration obtained by using the steady state current.

Referring to FIG.6, it is understood that ethanol gas exhibits a good response characteristic in the range between 3ppm and 600ppm and that the signals having no difficulty in making a module of the bio-sensor into a device, sensitivity and linear characteristics are excellent. The bio-sensor having excellent characteristics by completing the characteristic evaluation as described above is used for manufacturing a portable drunkometer.

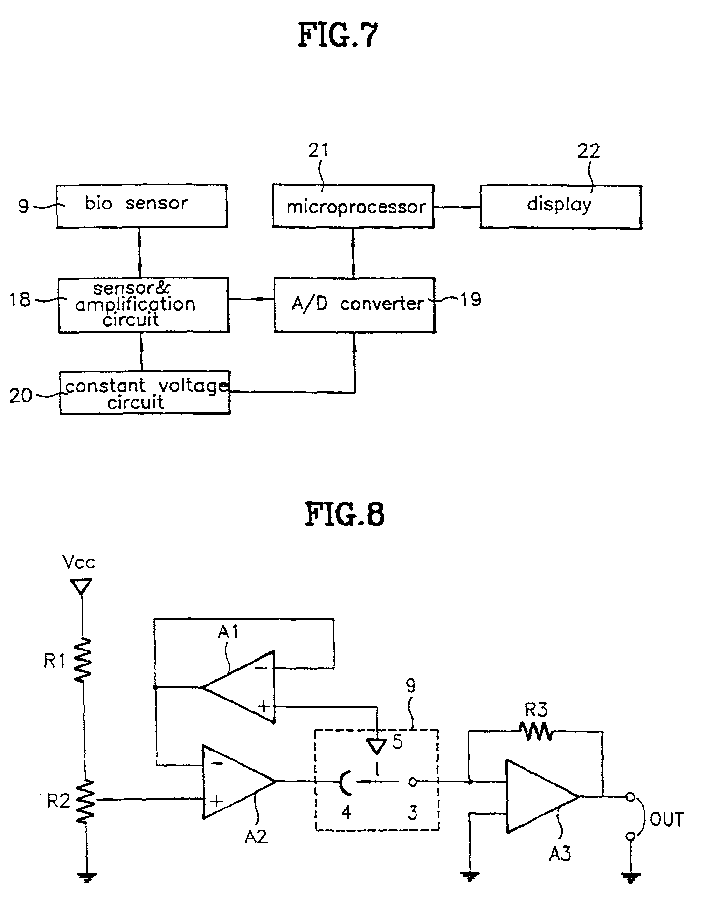

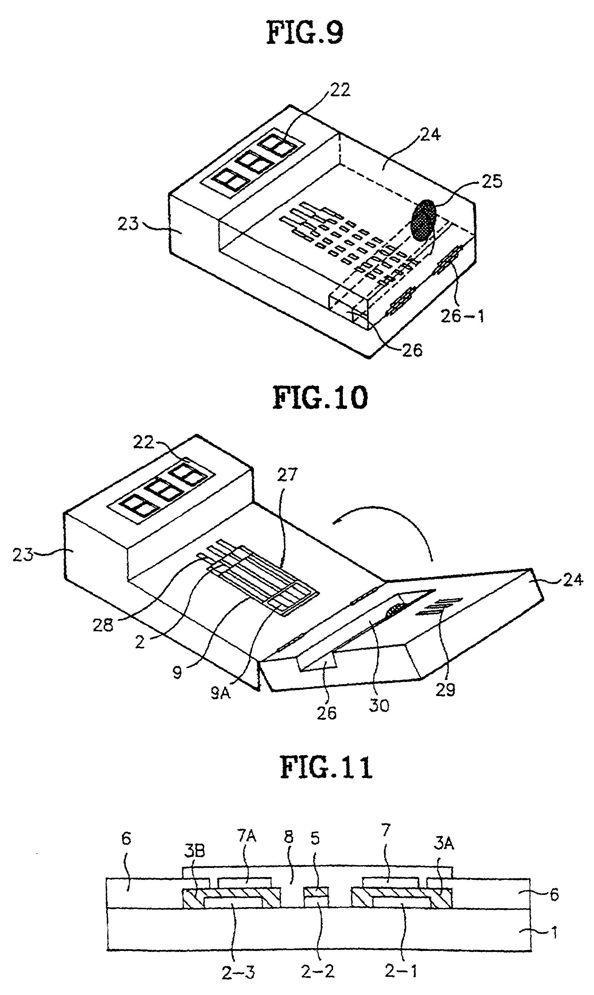

FIG.7 is a circuit diagram of a drunkometer, using a bio-sensor and embodying the present invention. FIG.8 is a detailed circuit diagram of the sensor & amplifying circuit shown in FIG.7, FIG.9 is a perspective view of the drunkometer, and FIG.10 is a perspective view of the drunkometer when the cover is opened.

Referring to FIG.7, the drunkometer includes a bio-sensor 9, a sensor & amplifying circuit 18 for sensing the current generated during reaction of bio-sensor 9 with alcohol gas and amplifying the same, an analog-to-digital converter 19 for converting the signal generated from sensor & amplifying circuit 18 into a digital signal, a constant voltage circuit 20 for adjusting the differential of electric potential of working electrode with respect to reference electrode on bio-sensor 9 through sensor & amplifying circuit 18, a microprocessor 21 for processing the digital signal output from analog-to-digital converter 19, converting and outputting the drinking degree, and a display 22 for displaying the drinking degree output from microprocessor 21 in a digital value.

The operation of the drunkometer having aforementioned configuration will now be described.

Constant voltage circuit 20 adjusts the differential of electric potential of working electrode 3 with respect to reference electrode of bio-sensor 9 so as to be 650mV. If doing so, bio-sensor reacts with alcohol gas in a testee's exhalation, the thus-generated NADH is oxidized to be NAD+ on working electrode 3, and current flows from working electrode 3 to counter electrode 4 at the same time. The current flowing from working electrode 3 to counter electrode 4 is sensed and amplified by sensor & amplifying circuit 18, and amplified signal is applied to analog-to-digital converter 19 to then be converted into a digital signal.

Analog-to-digital converter 19 converts the input signal for measuring the drinking degree to then apply the signal to microprocessor 21. Microprocessor 21 processes input digital signal for measuring the drinking degree. Display 22 inputs the processed signal from microprocessor 21 and displays ethanol concentration as a digital value.

The operation of sensing and amplifying the current flowing between electrodes of bio-sensor depending on the ethanol concentration will be described in detail with reference to the detailed diagram of sensor & amplifying circuit 18 shown in FIG.8.

In sensor & amplifying circuit 18, the constant voltage applied from constant voltage circuit 20 is distributed by resistance R1 to then be applied to a noninverted port (+) of an operational amplifier A2. The output signal of operational amplifier A2 is fed back via bio-sensor 9 and operational amplifier A1 to then be applied to an inverted port (-), thereby adjusting the differential of electric potential of working electrode 3 with respect to reference electrode of bio-sensor 9 to become 650mV. In the state where the differential of electric potential is adjusted, bio-sensor 9 reacts with alcohol gas contained in the human's exhalation. While the NADH generated in reacting with ethanol, is being oxidized to be NAD⁺ on working electrode 3, current flows from working electrode 3 to counter electrode 4. The current I flowing from working electrode 3 to counter electrode 4 is amplified via an operational amplified A3 depending on the gain determined by resistance R3 to then be output to an output port OUT.

The value of the current amplified and output from sensor & amplifying circuit 18 represents an ethanol concentration, i.e., a measured signal of the drinking degree. This signal is supplied to analog-to-digital converter 19 so that the drinking degree is displayed via display 22, as described above.

The example of realizing the drunkometer having aforementioned circuit configuration is described in FIGs.9 and 10.

Referring to FIG.9, the drunkometer according to the present invention is provided with a display 22 on its main body 23 and the display 22 is constituted by 7-segment display device for the easy identification of the drinking degree.

Also, in the main body 23 of the drunkometer, there is provided with a cover 24 being connected to the main body 23 by means of a hinge 26-1, which may be opened or shut. A sample gas inlet 25 exposed outwardly is formed on the whole surface of cover 24, thereby enabling to blow the testee's respired gas in measuring the drinking degree.

Sample gas outlet 26 is formed on the side of cover 24 for exhausting the remaining gas after the bio-sensor in the main body 23 of the drunkometer reacts with the exhaled gas blown through sample gas inlet 25.

FIG.10 is a perspective view of the drunkometer when its cover 24 is opened.

If the cover 24 is open, there is formed a bio-sensor placement portion 27 for placing bio-sensor 9 on the location shut by the cover 24 of the main body 23. On one side of bio-sensor placement portion 27, a bio-sensor connective pad 28 for electrically connecting with bio-sensor 9 is formed.

Another bio-sensor connective pad 29 contacting with bio-sensor connective pad 28 is inside the cover 24. When the cover is shut, two pads contact with each other, thereby turning on power switch of the drunkometer.

Also, sample gas path 30 for exhausting the human respired gas blown through sample gas inlet 25 is formed inside the cover 24. Sensitive portion 9A of bio-sensor 9 is located in about the middle of the sample gas path 30, thereby reacting with alcohol among the sample gases.

Therefore, when the drinking degree is measured by means of the drunkometer, individually packed bio-sensor is first opened and the cover 24 of the drunkometer is opened later to then place the opened bio-sensor 9 to bio-sensor placement portion 27. At this time, the placement is performed such that sensitive portion 9A of bio-sensor 9 is placed on sample gas path 30, to then shut cover 24.

If cover 24 is shut, the bio-sensor connective pad 29 installed on cover 24 contacts with the bio-sensor connective pad 28 being on main body 23 and the power switch of the drunkometer is turned on at the same time. That is to say, as shown in FIG.7, power is supplied from constant voltage circuit 20 so that the current reacting with alcohol gas of bio-sensor 9 becomes detectable.

In such a state, if the testee's exhaled gas is blown into sample gas inlet 25, the respired gas reacts with sensitive portion 9A of bio-sensor 9 while transmitting through sample gas path 30 and the remained gas is exhausted out via sample gas outlet 26.

The reaction of sensitive portion 9A generates the current proportionate to ethanol concentration on electrodes of bio-sensor 9, as described with reference to FIGs.7 and 8. The generated current is calculated to be the testee's drinking degree by various signal processes, as described above, to then be displayed in display 22. The measurer identifies the drinking degree displayed in display 22, thereby enabling to discern alcohol concentration.

FIG.11 is a constitutional diagram of another bio-sensor.

In this bio-sensor, two working electrodes 3A and 3B are formed, and response interference, i.e., temperature change except enzymatic reactions may be prevented by using a differential amplifier. In other words, in the biosensor shown in FIG.1, since the sizes of working electrode 3 and counter electrode 4 are the same, working electrode 3 and counter electrode 4 are used as two working electrodes 3A and 3B. Also, the value of current reacting with alcohol, shown on the respective electrode is detected by means of a differential amplifier. Thus, the causes of response interference, i.e., temperature change, other than enzymatic reactions, are prevented, and the sensitivity of output signals can be improved.

At this time, enzyme immobilized layer 7 is formed only on one working electrode 3A, and a layer from which enzyme activity is removed is printed and formed on the other working electrode 3B. Other processes are the same as those in the first embodiment.

Using the bio-sensor shown in Figure 11, the alcohol concentration contained in an aqueous solution, e.g., saliva or blood, can be measured.

However, when alcohol concentration in blood after drinking, the response interference may be caused by an electroactive material other than NADH, such as ascorbic acid present in blood. This is because NADH is oxidized at the differential of electric potential of 650mV, which is required for the oxidation of NADH.

To overcome such a phenomenon, electron mediator is absorbed on the carbon working electrode so that the electrochemical regeneration of NADH can be easily achieved, or is bonded with aqueous high polymers to then be used for printing enzyme immobilized layer, thereby enabling to operate electrodes at a low differential of electric potential. Thus, the response interference due to another electroactive materials present in blood can be prevented, and electricity consumption can be saved as well.

With the embodiments as described above, the below-mentioned effects can be achieved.

Firstly, a bio-sensor has been described which can measure alcohol concentration by reacting with vapor-phase alcohol, as well as a method for manufacturing the strip-type bio-sensor for measuring alcohol concentration, and a drunkometer using the bio-sensor for measuring alcohol concentration.

Secondly, since the drunkometer using the bio-sensor can measure the drinking degree precisely after drinking, the objectivity in measuring the drinking degree can be secured, compared to that using the conventional gas sensor.

Thirdly, since the whole processes for manufacturing the bio-sensor as described above, including enzyme immobilized layer can be performed utilizing those for forming an amperometric device, it is advantageous to produce a large volume of bio-sensors.

Fourthly, an electric system can be formed easily only with moisture contained in the sample gas in measuring gases. Also, it is possible for bio-sensors to react with a vapor-phase sample only on drinking by packing the bio-sensors individually. Thus, the stability for heat is improved and life span thereof becomes long. Also, the signal-to -noise becomes lesser than those for another bio-sensor used in an aqueous solution.

Fifthly, since a high enzyme affinity for a vapor-phase organochemical material can be used, it is possible to measure the drinking degree with a lower concentrated sample.

## Claims

1. A breath alcohol analyzer for measuring alcohol concentration comprising:
a biosensor (9) for reacting with a vapor-phase alcohol gas;
a sensor and amplifying circuit (18) for sensing and amplifying the current generated from the reaction of the biosensor with the alcohol gas;
an analog-to-digital converter (19) for converting a signal output from said sensor and amplifying circuit into a digital signal;
a microprocessor (21) for processing said digital signal output from said analog-to-digital converter and determining a drinking degree corresponding to the digital signal, the microprocessor outputting a signal indicating the drinking degree; and
a display (22) for receiving the signal output from the microprocessor and displaying the drinking degree.

2. A breath alcohol analyzer as claimed in claim 1, wherein the biosensor comprises:
an insulating substrate (1);
a thick film amperometric device formed on the insulating substrate and having a plurality of conductive lines and connective pads (2) and a plurality of electrodes (3-5);
an enzyme immobilized layer (7) formed on one of the plurality of electrodes of the amperometric device;
an outer membrane (18) formed on the insulating substrate having said plurality of electrodes for forming an electrode system; and
an insulating membrane (16) formed on said substrate except on the outer membrane.

3. A breath alcohol analyzer for measuring alcohol concentration as claimed in claim 2, wherein said plurality of electrodes are composed of a working electrode (3), a counter electrode (4) and a reference electrode (5).

4. A breath alcohol analyzer for measuring alcohol concentration as claimed in claim 2, wherein said plurality of electrodes are composed of two working electrodes (3) and a reference electrode (5).

5. A breath alcohol analyzer for measuring alcohol concentration as claimed in claim 2, wherein said enzyme immobilized layer (7) is composed of a high polymer material, an enzyme, and a cofactor.

6. A breath alcohol analyzer for measuring alcohol concentration as claimed in claim 5, wherein said high polymer material is composed of a carbon powder and hydroxyethylcellulose.

7. A breath alcohol analyzer for measuring alcohol concentration as claimed in claim 5, wherein said enzyme is an alcohol dehydrogenase.

8. A breath alcohol analyzer for measuring alcohol concentration as claimed in claim 5, wherein said cofactor is NAD⁺.

9. A breath alcohol analyzer for measuring alcohol concentration as claimed in claim 2, wherein said outer membrane is a hydroxyethylcellulose.

10. A breath alcohol analyzer as claimed in any preceding claim, further comprising:
a main body (23) in which said microprocessor (21) and bio-sensor (9) are installed;
a cover (24) connected with said main body by means of a hinge (26-1) which can be open or shut;
a bio-sensor placement portion (27) being opposed to said cover, for placing said bio-sensor to the inner surface of said main body;
a sample gas inlet (25) formed on the side surface of said cover for blowing a testee's exhalation therein;
a sample gas path (30) for guiding the gas passed through said sample gas inlet so as to pass the sensitive portion of said bio-sensor; and
a sample gas outlet (26) for discharging the remaining gas passed through said sample gas path.

11. A breath alcohol analyzer as claimed in claim 10, wherein a power switch is turned on, if said cover is shut, by means of a first bio-sensor connective pad formed on the inner surface of said cover making contact with a second bio-sensor connective pad formed on said main body, the first pad being opposed to the second pad.

## Patentansprüche

1. Atem-Alkohol-Analysator zum Messen von Alkohol-Konzentration, umfassend:
einen Biosensor (9) zum Reagieren mit einem Dampf-Phasen-Alkoholgas;
eine Sensor- und Verstärker-Schaltung (18) zum Sensieren und Verstärken des aus der Reaktion des Biosensors mit dem Alkoholgas erzeugten Stromes;
einen Analog-/Digital-Wandler (19) zum Umwandeln eines von der Sensor- und Verstärker-Schaltung ausgegebenen Signals in ein digitales Signal;
einen Mikroprozessor (21) zum Verarbeiten des von dem Analog-/Digital-Wandler ausgegebenen digitalen Signals und zum Bestimmen eines dem digitalen Signal entsprechenden Trink-Grades, wobei der Mikroprozessor ein Signal ausgibt, welches den Trink-Grad angibt; und
eine Anzeige (22) zum Empfangen des vom Mikroprozessor ausgegebenen Signals und zum Anzeigen des Trink-Grades.

2. Atem-Alkohol-Analysator nach Anspruch 1, worin der Biosensor umfaßt:
ein isolierendes Substrat (1);
eine Dickschicht-Amperometrie-Vorrichtung, welche auf dem isolierenden Substrat gebildet ist und eine Mehrzahl von Leiterbahnen und Anschlußflecken (2) und eine Mehrzahl von Elektroden (3 bis 5) aufweist;
eine Schicht (7) mit immobilisierten Enzymen, welche auf einer von der Mehrzahl der Elektroden der Amperometrie-Vorrichtung gebildet ist;
eine äußere Membran (18), welche auf dem isolierenden Substrat gebildet ist und die Mehrzahl von Elektroden zum Bilden eines Elektrodensystems aufweist; und
eine isolierende Membran (16), welche auf dem Substrat gebildet ist außer auf der äußeren Membran.

3. Atem-Alkohol-Analysator zum Messen der Alkohol-Konzentration nach Anspruch 2, worin die Mehrzahl von Elektroden zusammengesetzt ist aus einer Arbeitselektrode (3), einer Gegenelektrode (4) und einer Referenzelektrode (5).

4. Atem-Alkohol-Analysator zum Messen der Alkohol-Konzentration nach Anspruch 2, worin die Mehrzahl von Elektroden zusammengesetzt ist aus zwei Arbeitselektroden (3) und einer Referenzelektrode (5).

5. Atem-Alkohol-Analysator zum Messen der Alkohol-Konzentration nach Anspruch 2, worin die Schicht (7) mit immobilisierten Enzymen zusammengesetzt ist aus einem Hoch-Polymer-Material, einem Enzym und einem Ko-Faktor.

6. Atem-Alkohol-Analysator zum Messen der Alkohol-Konzentration nach Anspruch 5, worin das Hoch-Polymer-Material zusammengesetzt ist aus einem Kohlenstoff-Pulver und Hydroxyethyl-Zellulose.

7. Atem-Alkohol-Analysator zum Messen der Alkohol-Konzentration nach Anspruch 5, worin das Enzym Alkohol-Dehydrogenase ist.

8. Atem-Alkohol-Analysator zum Messen der Alkohol-Konzentration nach Anspruch 5, worin der Ko-Faktor NAD⁺ ist.

9. Atem-Alkohol-Analysator zum Messen der Alkohol-Konzentration nach Anspruch 2, worin die äußere Membran eine Hydroxyethyl-Zellulose ist.

10. Atem-Alkohol-Analysator nach einem der vorangehenden Ansprüche, ferner umfassend:
einen Haupt-Körper (23), in welchem der Mikroprozessor (21) und Bio-Sensor (9) installiert sind;
eine Abdeckung (24), welche mit dem Haupt-Körper mittels eines Scharniers (26-1) verbunden ist, und welche geöffnet oder geschlossen werden kann;
einen Bio-Sensor-Plazierungs-Bereich (27) gegenüber der Abdeckung, um den Bio-Sensor zu der Innenfläche des Haupt-Körpers hin zu plazieren;
einen Probengas-Einlaß (25), welcher auf der Seitenfläche der Abdeckung gebildet ist, damit eine Testperson darin hineinpusten kann;
einen Probengas-Weg (30) zum Führen des Gases, welches den Probengas-Einlaß passiert hat, derart, daß es den meßwirksamen Bereich des Bio-Sensors passiert; und
einen Probengas-Auslaß (26) zum Ausstoßen des Restgases durch den Probengas-Weg.

11. Atem-Alkohol-Analysator nach Anspruch 10, worin ein Leistungsschalter angeschaltet ist, falls die Abdeckung geschlossen ist, mittels eines ersten Bio-Sensor-Anschlußfleckes, welcher auf der Innenfläche der Abdeckung gebildet ist und einen Kontakt herstellt mit einem zweiten Bio-Sensor-Anschlußfleck, welcher an dem Haupt-Körper gebildet ist, wobei sich der erste Anschlußfleck gegenüber dem zweiten Anschlußfleck befindet.

## Revendications

1. Analyseur d'alcoolémie pour mesurer la concentration en alcool, comportant :
un biocapteur (9) pour réagir avec un alcool gazeux en phase vapeur,
un circuit capteur et d'amplification (18) pour détecter et amplifier le courant généré par la réaction du biocapteur avec l'alcool gazeux,
un convertisseur analogique-numérique (19) pour convertir un signal de sortie dudit circuit capteur et d'amplification en un signal numérique,
un microprocesseur (21) pour traiter ledit signal numérique de sortie dudit convertisseur analogique-numérique et déterminer un degré d'alcoolémie correspondant au signal numérique, le microprocesseur émettant un signal indiquant le degré d'alcoolémie, et
un affichage (22) pour recevoir le signal de sortie du microprocesseur et afficher le degré d'alcoolémie.

2. Analyseur d'alcoolémie selon la revendication 1, dans lequel le biocapteur comporte :
un substrat isolant (1),
un dispositif ampérométrique à film épais formé sur le substrat isolant et ayant une pluralité de lignes conductrices et de plages de connexion (2) et une pluralité d'électrodes (3-5),
une couche immobilisée enzymatique (7) formée sur une électrode de la pluralité d'électrodes du dispositif ampérométrique,
une membrane extérieure (18) formée sur le substrat isolant ayant ladite pluralité d'électrodes pour former un système d'électrodes, et
une membrane isolante (16) formée sur ledit substrat à l'exception de la membrane extérieure.

3. Analyseur d'alcoolémie pour mesurer la concentration en alcool selon la revendication 2, dans lequel ladite pluralité d'électrodes est constituée d'électrodes coportant une électrode de travail (3), une contre-électrode (4) et une électrode de référence (5).

4. Analyseur d'alcoolémie pour mesurer la concentration en alcool selon la revendication 2, dans lequel ladite pluralité d'électrodes est constituée d'électrodes comportant deux électrodes de travail (3) et d'une électrode de référence (5).

5. Analyseur d'alcoolémie pour mesurer la concentration en alcool selon la revendication 2, dans lequel ladite couche immobilisée enzymatique (7) est constituée d'un matériau polymère supérieur, d'une enzyme et d'un cofacteur.

6. Analyseur d'alcoolémie pour mesurer la concentration en alcool selon la revendication 5, dans lequel ledit matériau en polymère supérieur est constitué d'une poudre de carbone et d'hydroxyéthylcellulose.

7. Analyseur d'alcoolémie pour mesurer la concentration en alcool selon la revendication 5, dans lequel ladite enzyme est un alcool-déshydrogénase.

8. Analyseur d'alcoolémie pour mesurer la concentration en alcool selon la revendication 5, dans lequel ledit cofacteur est NADPH⁺.

9. Analyseur d'alcoolémie pour mesurer la concentration en alcool selon la revendication 2, dans lequel ladite membrane extérieure est une hydroxyéthylcellulose.

10. Analyseur d'alcoolémie selon l'une quelconque des revendications précédentes, comportant de plus :
un corps principal (23) dans lequel sont installés ledit microprocesseur (21) et ledit biocapteur (9),
un couvercle (24) connecté audit corps principal par l'intermédiaire d'une articulation (26-1) qui peut être ouverte ou fermée,
une partie de placement de biocapteur (27) étant opposée audit couvercle, pour placer ledit biocapteur sur la surface intérieure dudit corps principal,
une entrée d'échantillon gazeux (25) formée sur la surface latérale dudit couvercle pour souffler une exhalation de test à l'intérieur,
un trajet d'échantillon gazeux (30) pour guider le gaz passé à travers ladite entrée d'échantillon gazeux de manière à faire passer la partie sensible dudit biocapteur, et
une sortie d'échantillon gazeux (26) pour décharger le gaz restant passé à travers ledit trajet d'échantillon gazeux.

11. Analyseur d'alcoolémie selon la revendication 10, dans lequel un interrupteur est rendu passant, si ledit couvercle est fermé, par l'intermédiaire d'une première plage de connexion de biocapteur formée sur la surface intérieure dudit couvercle établissant un contact avec une seconde plage de connexion de biocapteur formée sur ledit corps principal, la première plage étant opposée à la seconde plage.
